# EUROPEAN PATENT SPECIFICATION

(11) **EP 0 356 580 B1**
(45) Date of publication and mention of the grant of the patent: **07.06.1995**
(21) Application number: 88119875.8
(22) Date of filing: 29.11.1988
(51) Int. Cl.: C08J 7/12, A61L 31/00

(54) **Method for treating the surface of powderfree surgical gloves**
Verfahren zur Oberflächenbehandlung von puderfreien Operationshandschuhen
Procédé pour le traitement de surface de gants chirurgicaux exempts de poudre

(30) Priority: 19.08.1988 JP 205980/88
(43) Date of publication of application: 07.03.1990
(73) Proprietor: ZAIDANHOJIN RINSHOGANKA KENKYUJO(Institute of Clinical Ophthalmology), Japan 376-06 (JP)
(72) Inventor: Momose, Akira, 1-100 Umeda Kiryu Gunma 376-06 (JP); Nakano, Fumihiko, Tochigi 321-43 (JP)
(74) Representative: Reinhard - Skuhra - Weise & Partner

(56) References cited:
- EP-A- 0 071 587
- WO-A-83/03421
- US-A- 4 597 108
- DATABASE (DERWENT) WPI, accession no. 80-17998C [10], Derwent Publications Ltd, London, GB

## Description

The present invention relates to a method for treating the surfaces of natural rubber gloves used during surgical procedures and more specifically, to a method for treating the surfaces of a natural rubber gloves in a process for producing natural rubber gloves which are excellent in wearing and in handling of surgical instruments or tools during operative procedures. That is, in said method, the inner and the outer surfaces of the gloves are halogenated to different degrees in order to impart different degrees of slipperiness to the inner surface and the outer surface without the need of powders or exterior finishes.

It has long been recognized that natural rubber in the form of pure latex produced the best gloves for the surgeon in terms of wearing comfort and tactile sensitivity.

However, untreated natural latex rubber gloves produced by the conventional methods have posed such problems that the wearing comfortability thereof has become worse due to the facts that when the gloves are left as they are, they are conglutinated in their inside and that they tend to stick to the fingers or hands during the insertion process as well as the less slipperiness on the outer surface of such gloves causes a surgeon donning or wearing the gloves not to smoothly handle surgical instruments such as pinsets, scapels, etc. can not be used completely.

For solving the problems, the prior art has proposed the use of various kinds of powders, such as talcum powder, corn starch and other proprietary powders that have the effect of lubricating the inside and outside of the gloves, thereby enabling the surgeon to insert his hands into the gloves without difficulty.

However, recent experimentations have shown that the various lubricating powders used on surgical gloves have a deleterious effect in contamination to surgical wound by the powders is possible and that in many cases serious complications have actually resulted from such contamination, which are, of course, harmful to the patient. Moreover, there have been increased operating rooms in a type of bioclean rooms and therefore, these powders have been recognized to be one of the causes which pollute the operating room.

Furthermore, the dusting of the gloves with these kinds of lubricating powders could not be limited to the inside of the gloves. Powders must be applied to the outer surface of the gloves as well to prevent adhesion of latex to adjoining latex, unless the outer surface is treated somehow to make it non-sticky. The application of these lubricating powders as mentioned above has such drawbacks that much of them are actually retained on the outer surface of the glove even though the gloves after the insertion are washed to wash out such powders; spilling out of the powder from the inside to the outside of a glove can occur when a pinhole is made in the glove by injection needle during the surgical operation or when the glove is torn during use in the surgical operation; and the lubricating powder provided inside of the glove come out of the outer surface through the sleeve of the glove. Thus, a powderfree glove for surgical operation has been expected, in which slipperiness of the inner and outer surfaces of the glove is improved.

The problems associated with contamination resulting from cornstarch, talcum powder and the like have been commented upon in an article entitled "Powderfree Surgical Gloves", appearing in a magazine entitled "Ophthalmic Surgery", by Villasenor, Harris, Barron, Krasnow and Salz, of the Estelle Doheny Eye Foundation, University of Southern California School of Medicine, Los Angeles, California. This article is cited primarily for its extensive bibliography concerning the contamination resulting from lubricating powders used on surgical gloves.

The prior art recoginized these problems and attempted to solve them by making both the outside and the inside of the gloves more slippery by means of a halogenation process. These attempts were successful in varying degrees in making it easier to don the gloves. However, the halogenation process had the unfortunate side effect of making the outer surface of the gloves too slippery for the surgeon to firmly hold and manipulate the instruments in his hands. Moreover, the halogenation process, unless precisely controlled, frequently resulted in excessive halogenation and deterioration of the latex, reducing the mechanical strength of the gloves.

The prior art attempted to overcome this difficulty by means of limited halogenation of both the outer and inner surfaces of the gloves combined with the use of powder on the inside of the gloves for the purpose of additional lubrication.

A review of the art shows that U.S.P. 3,411,982, entitled "Elastomeric Article Having A Slip Coating" by J. J. Kavalir et al., discloses the process of halogenating the surface of rubber gloves with bromine or chlorine to make it slippery. The U.S.P. 3,411,982 discloses primarily to show other solutions of utilizing a halogenation process in developing a slip coating to achieve the desired slipperiness of the outer surface of the gloves.

U.S.P. 3,740,262, entitled "Dual Finish Surgeon's Gloves and Method of Making Same" and issued to A. J. Agostinelli, also teaches the treatment of a surface of a surgeon's glove with any halogen, preferably with chlorine or bromine.

Interestingly, the U.S.P. 3,740,262 recognizes that treating the inside of the glove with the halogenation process makes the glove too slippery and hence unacceptable and, here again, said patent discloses the use of suitable lubricating powders to solve the insertion problem. The U.S.P. 3,740,262 is interesting for the complete disclosure of the problems associated with utilizing a surgeon's glove and does disclose many procedures for minimizing the effect of the powder used to minimize patient contamination by the powder.

U.S.P. 3,967,014, entitled "Rubber Articles Having Improved Slip Coating" and issued to P. E. Esemplare et. al., also discloses the concept of applying a slip coating on the outer surface of rubber gloves as a means of controlling the slipperiness on the outer surface of the gloves. The U.S.P. 3,967,014 shows the development of the art in minimizing the use of lubricating powders on surgical gloves.

U.S.P. 3,992,221, entitled "Method of Treating Extensible Hydrocarbon Articles" and issued to Homsy et. al., discloses a preferred method of halogenating the outer surface of a surgeon's glove. The U.S.P. 3,992,221 discloses certain advantages of utilizing chlorine gas during the halogenation process as opposed to fluorine or other halogens.

The U.S.P. 3,992,221 does show and describe a glove treated on the outer surface to make it non-sticky so that powder is not needed to prevent the adhesion of latex. The U.S.P. 3,992,221 does treat the inner surface, but, interestingly, it does not disclose the concept of controlled outer surface treatment to obtain a degree of slipperiness that eliminates the need for powder. The U.S.P. 3,992,221 discloses only the treating of the outer surface of the glove.

U.S.P. 4,597,108, entitled "Powderfree Surgical Gloves" and issued to Akira Momose who is also the co-inventor of the present application, describes a preferred method for producing a surgeon's glove from pure latex rubber that is treated on both the outer and the inner surfaces to different degrees of halogenation to thereby obtain a desired inner surface frictional coefficient which is different from the outer surface frictional coefficient.

By controlling the halogenation process to obtain different degrees of slipperiness, U.S.P. 4,597,108 discloses how to produce a glove having a desired degree of slipperiness on the inner surface that allows the surgeon to insert his hands without difficulty and at the same time, having a different degree of slipperiness on the outer surface to thereby enhance the surgeon's ability to handle the necessary surgical instruments with minimum difficulty.

The prior art methods of producing chlorinated water generally include one of the following three halogenation methods or method according to these methods:
1. The direct injection of chlorine gas into the water mixture.
2. Mixing high density bleaching powder and aluminum chloride in water.
3. Brine electrolysis to produce chlorinated water.

However, the prior art of producing chlorinated water resulted in the generation of chlorine gas that is released into the air in excessive quantities and in a manner that is not tightly controlled in the area of the gloves being processed.

The prior art methods have not provide the required tight control and therefore resulted in products that are less than satisfactory from a quality control standpoint. The cost of production is unnecessarily high due to the high scrap rate entailed by the lack of control over the halogenation process.

It is one object of the present invention to provide a new and improved method of halogenating natural rubber surgical gloves.

According to the present invention there is provided a method of halogenating natural rubber surgical gloves on both the inner and outer surfaces to different degrees comprising the steps of:
- fitting the natural rubber glove over a hand-shaped mold,
- dipping the glove for 1 to 60 seconds in a sodium hypochlorite solution, wherein the concentration of effective chlorine in the solution is within the range of 0.1 to 15 %,
- dipping the glove in a hydrochloric acid solution for 1 to 60 seconds, wherein the concentration of effective chlorine in the solution is within the range of 0.1 - 15 %,
- washing the glove with water until all chlorine residue is removed,
- removing the glove from the mold and turning the glove inside out and fitting glove over the hand-shaped mold,
- dipping the glove for 1 to 60 seconds in a sodium hypochlorite solution, wherein the concentration of effective chlorine in the solution is within the range of 1 to 15 %,
- dipping the glove in a hydrochloric acid solution for 1 to 60 seconds, wherein the concentration of effective chlorine in the solution is within the range of 1 - 36 %,
- repeating the last-mentioned two steps at least 3 times to obtain a different degree of halogenation on the inner surface from that obtained on the outer surface, and
- washing the glove in water until all chlorine residue is removed.

In the process of the present invention, there is, therefore, described an improved method of producing chlorinated water that results in less free chlorine gas being released into the air in which the chemical reaction generates chlorine gas in largely localized areas close to the glove surface being processed. This allows a higher measure of control over the halogenation of the glove surface, whether it be the inner surface or the outer surface that is being processed, thereby resulting in a product having a higher quality control and less waste than in the prior art methods.

The improved method of generating chlorine gas and halogenating the gloves on the outer surface to a degree different from the halogenation of the inner surface is as follows:
In order to obtain glove having excellent handleability, the glove constructed of latex is fitted over a hand-shaped mold with the outer surface on the outside. The glove is then immersed for approximately 8 to 10 seconds in a sodium hypochlorite solution containing 1.5% effective chlorine. Immediately following the immersion, the glove is removed and then immersed for 8 to 10 seconds in a 0.16% hydrochloric acid solution after which the glove is removed, washed and dried.

After the treatment of the outer surface, in order to obtain the glove having excellent insertability, it is removed from the mold and turned inside out and refitted over the mold. The glove is then immersed for approximately 8 to 10 seconds in a sodium hypochlorite solution containing 6% effective chlorine. Immediately following this immersion, the glove is immersed for 8 to 10 seconds in a 12% hydrochloric acid solution. This treatment of the inner surface is repeated 3 or more times, depending on the degree of slipperiness required for the inner surface.

The described process imparts different degrees of slipperiness to the outer and inner surfaces of the glove and, because the chemical reaction generating chlorine gas is largely localized to the glove surface area, there is a greater control over the halogenation process, resulting in a greater efficiency.

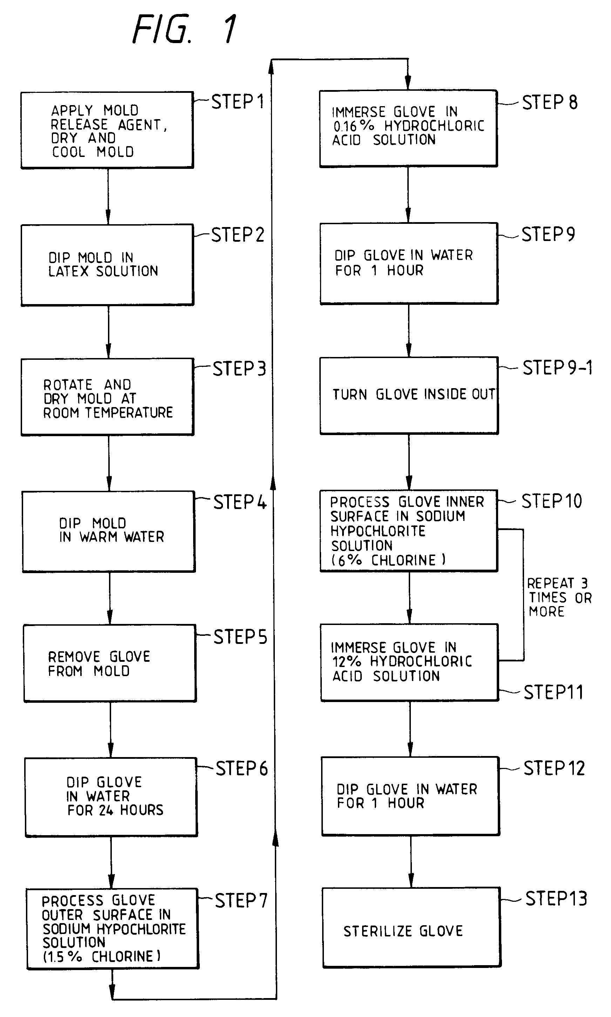

Further objects and advantages of the present invention will be made more apparent by referring to the attached drawing which illustrates a flow chart of the preferred method of processing both the inner and outer surface of the glove.

The present invention is concerned primarily and uniquely with the production of an improved surgical natural rubber glove that completely and once for all eliminates the need for powder of any kind.

In the practice of the present invention, both the inner and outer surfaces of the natural latex rubber glove are treated with chlorine during the halogenation process, but are halogenated to different degrees so as to differentiate the degree of slipperiness between the outer and inner surfaces.

It is thus possible to have a halogenated latex rubber glove that preserves the external tactile feeling needed by the surgeon to securely hold his instruments, together with the ease of use that makes it convenient for the surgeon to insert his hand into the gloves without the need of powder of any kind.

In the practice of the present invention, the outer surface of the glove is treated with less chlorine and the inner surface with more chlorine when manufacturing the glove, in order to enable the fingers and hands to be inserted easily into the glove and still allow the surgical instruments to be held firmly.

In the manufacture of the glove, a mold is chosen that fits the shape of the surgeon's hand and the glove is made with both the back and the palm of the hand accentuated, thereby allowing the glove to respond freely with the bending of the fingers during use.

The main invention covers the method of manufacturing the surgical gloves by dipping a natural rubber glove in a sodium hypochlorite solution which contains 1.5% effective chlorine for a period of time ranging from 8 to 10 seconds after which the glove is then dipped in a 0.16% hydrochloric acid solution for a period of time ranging from 8 to 10 seconds.

Then, in treating the inner surface, the glove is turned inside out and fitted over the mold as previously described and then dipped in a sodium hypochlorite solution which contains 6% effective chlorine for a period of time ranging 8 to 10 seconds. The glove is then dipped in a 12% hydrochloric acid solution for a period of time ranging from 8 to 10 seconds. This step is preferably repeated 3 or more times, depending upon the degree of slipperiness required. The differential chlorinating process described imparts different digrees of slipperiness to the outer and inner surface.

Referring now to the accompanying diagram, there will be described in more detail an example of an actual test conducted on the method of manufacturing surgical gloves treated with chlorine based upon the teachings of the present invention in which the individual steps have been identified steps in the accompanying drawing.

According to the method of the present invention, in the step that the glove is immersed in the hydrochloric acid solution, sodium hypochlorite adhered to the surface of the glove brings about decomposition reaction to generate chlorine gas on the surface of the glove. The generation amount of the chlorine gas depends on the amount of sodium hypochlorite and pH. That is, the sodium hypochlorite is stable at the side of alkali, but brings about the decomposition at the neutral range. In this situation, since the step in which the inside of the glove is treated has higher concentration of sodium hypochlorite and lower pH than the step in which the outside of the glove is treated, much more chlorine gas generates and therefore, the inside surface of the glove can be treated more smoothly in comparison with the outside surface of the glove. As mentioned above, moreover, the chlorine gas generates from sodium hypochlorite adhered to the surface of the glove in the hydrochloric acid solution in either of the cases where the inside and outside of the glove are surface-treated. Since the place where the chlorine gas is thus generated is greatly localized, halogenation procedure can be more strictly controlled, whereby the quality of the glove can be improved.

In the diagram, there is shown a flow diagram comprising a plurality of steps for processing latex rubber gloves according to the teachings of the present invention to produce a powderfree surgical glove having a halogenated outer surface that is different from the halogenated inner surface.
- STEP 1:: When making powderfree surgical gloves from pure natural rubber (latex), the hand-shaped mold made of a ceramic material is dipped in a solution containing calcium nitrate or calcium chloride. This step is necessary to allow the natural rubber gloves to be peeled off the hand-shaped mold.
- STEP 2:: The hand-shaped mold is dipped in a solution containing 40% pure natural rubber (latex). A very small amount of ammonium chloride is added to this pure natural rubber solution to act as a stabilizer.
- STEP 3:: The hand-shaped mold is pulled out of the pure natural rubber solution and the mold is turned over many times in order to produce a uniform thickness of the rubber. The glove is then placed in a stationary position with the fingers pointing upwards. This is done for the purpose of making the finger portions of the glove relatively thin on the order of 200 microns, while a palm portion or the palm and back portions of the glove is relatively thick. The glove is then dried in this position.
- STEP 4:: The natural rubber adheres to the hand-shaped mold and after the rubber is dried to obtain the rubber glove, the rubber glove together with the hand-shaped mold is dipped in warm water.
- STEP 5:: The natural rubber becomes slightly soft from the warm water which then allows the glove to be peeled off from the mold.
- STEP 6:: The glove is again dipped in water and washed with water for the purpose of removing the calcium nitrate or calcium chloride that was used as set forth in STEP 1.
- STEP 7:: The glove is dipped in a sodium hypochlorite solution containing 1.5% effective chlorine for approximately 8 to 10 seconds.
- STEP 8:: The glove is then immediately immersed in a 0.16% hydrochloric acid solution for approximately 8 to 10 seconds.
- STEP 9:: The glove is washed in water for approximately one hour to remove all chlorine residue.
- STEP 10:: The glove is then turned inside out and refitted over the mold and again immersed in a sodium hypochlorite solution containing 6% effective chlorine for approximately 8 to 10 seconds.
- STEP 11:: The glove is then immediately immersed in a 12% hydrochloric acid solution for approximately 8 to 10 seconds. This treatment of the inner surface as set forth in STEP 10 and STEP 11 is repeated at least 3 times, depending upon the degree of slipperiness to be achieved.
- STEP 12:: The glove is washed in water one more time for approximately one hour to remove all chlorine residue.
- STEP 13:: The glove is dried and then sterillized, preferably with ethylene oxide gas (EOG).

By changing the shape of the mold in STEP 1 can be suitably provided powderfree surgical gloves of various sizes of inch for surgeons who have various sizes of, e.g., 6, 6 and 1/2, 7, 7 and 1/2, 8 inches and so on. By the STEP 3, it is produced a powderfree surgical glove having the palm portion or the palm and back portions of the glove being thickened in thickness, resulting in tightness; on the other hand, finger portions being thin and freely bendable. In STEPs 8 and 9 and STEPs 10 and 11, the outer surface of the glove is less halogenated, while the inner surface thereof is much halogenated. According to this degree of the halogenation, a powderfree surgical glove can be produced, in which slipperiness of the both inner and outer surfaces of the glove is suitably increased.

Thus, natural rubber surgical gloves (powderfree sergical glove) produced by the method described allow the surgeon to more easily insert his hands into them, while at the same time providing the tactile feeling so important to precisely holding and controlling surgical instruments.

The concentrations of sodium hypochlorite solution and hydrochloric acid in the above producing steps may be changed depending on the reaction such as time, temperature, etc. For example, the concentration of effective chlorine in the sodium hypochlorite solution in STEP 7 may be changed within the range of 0.1 - 15%, preferably 0.5 - 5%. In STEP 10, the concentration of effective chlorine in the sodium hypochlorite solution may be changed within the range of 1 - 15%. The concentrations of effective chlorine in hydrochloric acid solution in STEPs 8 and 11 may be changed in dependence of the amounts of hydrochloric acid solution to be used and of sodium hypochlorite which is taken into the hydrochloric acid solution, so that there is no specific limitation thereof. However, in the case that fair amount of hydrochloric acid solution exists in comparison with the amount of sodium hypochlorite which is taken into, e.g., when the surgical gloves are industrially manufactured, the concentration of effective chlorine may be preferably changed within the range of 0.1 - 15% in the STEP 8. On the other hand, in STEP 11, such a concentration may be changed within the scope of 1 - 36%, preferably 6 - 24%.

The immersion time of the glove in the solution in STEPs 7, 8, 10 and 11 may be, respectively,1 - 60 seconds. However, it is not appropriate to spend more than 30 seconds in one step of conveying operation of gloves in view of cost. Accordingly, it is preferable to be less than 30 seconds, especially 8 - 10 seconds.

Moreover, the concentrations of effective chlorine in sodium hypochlorite solution in STEPs 7 and 10 and the concentration of hydrochloric acid in the hydrochloric acid solution and immersion time of glove in STEPs 8 and 11 may be adopted as standards. For example, when the concentrations of effective chlorine in sodium hypochlorite solutions in STEPs 7 and 10 are respectively raised more than 1.5% and 6% of the Example, it is to be so suitably adjusted that halogenation is conducted outer and inner surfaces of the glove by the arrangement that the concentration of hydrochloric acid in the hydrochloric acid solution in STEP 8 is to be more than 0.16%; pH is retained within the range where sodium hypochlorite conducts decomposition reaction to generate chlorine gas; the concentration of hydrochloric acid in hydrochloric acid solution in STEP 11 is retained at 12%; and the immersion time of glove in hydrochloric acid solution in STEPs 8 and 10 is shortened than 8 - 10 seconds.

As explained above, according to the present invention, sodium hypochlorite adhered to the surface of the glove becomes at first neutral or acidic in pH to thereby generate chlorine gas, so that it is easy to control the amount of chlorine gas. Moreover, since the generated chlorine gas are largely localized on the surface of the glove, the chlorine gas can be effectively utilized for halogenation on the glove surfaces. Accordingly, the halogenation degree of the glove surfaces can be strictly controlled in comparison with the conventional methods, whereby effectively improving the producing ratio of good products in manufacture of the glove.

Furthermore, according to the present method, the inner surface of the glove can be halogenated in higher degree than the outer surface thereof and suitable slipperiness are given to the outer and inner surfaces according to their respective objects, the surgical gloves obtained from the present invention are excellent in both insertablity and handleability of surgical instruments.

## Claims

1. A method of halogenating natural rubber surgical gloves on both the inner and outer surfaces to different degrees comprising the steps of:
- fitting the natural rubber glove over a hand-shaped mold,
- dipping the glove for 1 to 60 seconds in a sodium hypochlorite solution, wherein the concentration of effective chlorine in the solution is within the range of 0.1 to 15 %,
- dipping the glove in a hydrochloric acid solution for 1 to 60 seconds, wherein the concentration of effective chlorine in the solution is within the range of 0.1 - 15 %,
- washing the glove with water until all chlorine residue is removed,
- removing the glove from the mold and turning the glove inside out and fitting glove over the hand-shaped mold,
- dipping the glove for 1 to 60 seconds in a sodium hypochlorite solution, wherein the concentration of effective chlorine in the solution is within the range of 1 to 15 %,
- dipping the glove in a hydrochloric acid solution for 1 to 60 seconds, wherein the concentration of effective chlorine in the solution is within the range of 1 - 36 %, repeating the last-mentioned two steps at least 3 times to obtain a different degree of halogenation on the inner surface from that obtained on the outer surface, and
- washing the glove in water until all chlorine residue is removed.

2. A method of halogenating natural rubber surgical gloves on both the inner and outer surfaces to different degrees according to claim 1, comprising the steps of:
- fitting the natural rubber glove over a hand-shaped mold,
- dipping the glove for 8 to 10 seconds in a sodium hypochlorite solution, wherein the concentration of effective chlorine in the solution is within the range of 0.5 to 5 %, preferably 1.5 %,
- dipping the glove in a hydrochloric acid solution for 8 to 10 seconds, wherein the concentration of effective chlorine in the solution is within the range of 0.1 - 15%, preferably 0.16%,
- washing the glove with water until all chlorine residue is removed,
- removing the glove from the mold and turning the glove inside out and fitting glove over the hand-shaped mold,
- dipping the glove for 8 to 10 seconds in a sodium hypochlorite solution, wherein the concentration of effective chlorine in the solution is within the range of 1 to 15 %, preferably 6 %,
- dipping the glove in a hydrochloric acid solution for 8 to 10 seconds, wherein the concentration of effective chlorine in the solution is within the range of 6 - 24 %, preferably 12 %,
- repeating the last-mentioned two steps at least 3 times to obtain a different degree of halogenation on the inner surface from that obtained on the outer surface, and
- washing the glove in water until all chlorine residue is removed.

3. A method of halogenating natural rubber surgical gloves on the outer surface comprising the steps of:
- fitting the natural rubber glove over an appropriate handshaped mold,
- dipping the glove in a sodium hypochlorite solution for 8 to 10 seconds, wherein the concentration of effective chlorine in the solution is 1.5 %,
- dipping the glove in a hydrochloric acid solution for 8 to 10 seconds, wherein the concentration of effective chlorine in the solution is 0.16 %, and
- washing the glove in water until all chlorine residue is removed.

4. A method of halogenating natural rubber surgical gloves on the inner surface comprising the steps of:
- first turning the natural rubber glove inside out and fitting the glove over an appropriate hand-shaped mold,
- dipping the glove in a sodium hypochlorite solution for 8 to 10 seconds, wherein the concentration of effective chlorine is 6 %,
- dipping the glove in a hydrochloric acid solution for 8 to 10 seconds, wherein the concentration of effective chlorine is 12 %,
- repeating the last-mentioned two steps at least 3 times to obtain a desired degree of halogenation on the inner surface, and
- washing the glove in water until all chlorine residue is removed.

## Patentansprüche

1. Verfahren zur Halogenierung chirurgischer Handschuhe aus natürlichem Gummi sowohl auf den Innen- als auch Außenflächen in unterschiedlichem Ausmaß mit den Schritten:
- Aufziehen des Handschuhs aus natürlichem Gummi über eine handförmige Form,
- Eintauchen des Handschuhs für 1 bis 60 Sek. in eine Natriumhypochloritlösung, wobei die Konzentration an wirksamem Chlor in der Lösung im Bereich von 0,1 bis 15% liegt,
- Eintauchen des Handschuhs in eine Salzsäurelösung für 1 bis 60 Sek., wobei die Konzentration an wirksamem Chlor in der Lösung im Bereich von 0,1 bis 15% liegt,
- Waschen des Handschuhs mit Wasser, bis alle Chlorreste entfernt sind,
- Entfernen des Handschuhs von der Form und Umdrehen des Handschuhs und Aufziehen des Handschuhs über die handförmige Form,
- Eintauchen des Handschuhs für 1 bis 60 Sek. in eine Natriumhypchloritlösung, wobei die Konzentration an wirksamem Chlor in der Lösung im Bereich von 1 bis 15% liegt,
- Eintauchen des Handschuhs in eine Salzsäurelösung für 1 bis 60 Sek., wobei die Konzentration an wirksamem Chlor in der Lösung im Bereich von 1 bis 36% liegt,
- wenigstens dreimaliges Wiederholen der zuletzt genannten zwei Schritte, um einen unterschiedlichen Halogenierungsgrad auf der Innenfläche im Vergleich zu der Außenfläche zu erhalten, und
- Waschen des Handschuhs in Wasser, bis alle Chlorreste entfernt sind.

2. Verfahren zum Halogenieren von chirurgischen Handschuhen aus natürlichem Gummi sowohl auf der Innen- als auch auf der Außenfläche in unterschiedlichem Ausmaß nach Anspruch 1 mit den Schritten:
- Aufziehen des Handschuhs aus natürlichem Gummi über eine handförmige Form,
- Eintauchen des Handschuhs für 8 bis 10 Sek. in eine Natriumhypochloritlösung, wobei die Konzentration an wirksamem Chlor in der Lösung im Bereich von 0,5 bis 5%, bevorzugt bei 1,5%, liegt,
- Eintauchen des Handschuhs in eine Salzsäurelösung für 8 bis 10 Sek., wobei die Konzentration an wirksamem Chlor in der Lösung im Bereich von 0,1 bis 15%, bevorzugt bei 0,16%, liegt,
- Waschen des Handschuhs mit Wasser, bis alle Chlorreste entfernt sind,
- Entfernen des Handschuhs von der Form und Umdrehen des Handschuhs und Aufziehen des Handschuhs über die handförmige Form,
- Eintauchen des Handschuhs für 8 bis 10 Sek. in eine Natriumhypchloritlösung, wobei die Konzentration an wirksamem Chlor in der Lösung im Bereich von 1 bis 15%, bevorzugt bei 6%, liegt,
- Eintauchen des Handschuhs in eine Salzsäurelösung für 8 bis 10 Sek., wobei die Konzentration an wirksamem Chlor in der Lösung im Bereich von 6 bis 24%, bevorzugt bei 2%, liegt,
- wenigstens dreimaliges Wiederholen der zuletzt genannten zwei Schritte, um einen unterschiedlichen Halogenierungsgrad auf der Innenfläche im Vergleich zu der Außenfläche zu erhalten, und
- Waschen des Handschuhs in Wasser, bis alle Chlorreste entfernt sind.

3. Verfahren zum Halogenieren von chirurgischen Handschuhen aus natürlichem Gummi auf der Außenfläche mit den Schritten:
- Aufziehen des Handschuhs auf natürlichem Gummi über eine geeignete handförmige Form,
- Eintauchen des Handschuhs in eine Natriumhypochloritlösung für 8 bis 10 Sek., wobei die Konzentration an wirksamem Chlor in der Lösung 1,5% beträgt,
- Eintauchen des Handschuhs in eine Salzsäurelösung für 8 bis 10 Sek., wobei die Konzentration an wirksamem Chlor in der Lösung 0,16% beträgt, und
- Waschen des Handschuhs in Wasser, bis alle Chlorreste entfernt sind.

4. Verfahren zum Halogenieren von chirurgischen Handschuhen aus natürlichem Gummi auf der Innenfläche mit den Schritten:
- Umdrehen des Handschuhs aus natürlichem Gummi, so daß die Innenseite nach außen zeigt, und Aufziehen des Handschuhs über eine geeignete handförmige Form,
- Eintauchen des Handschuhs in eine Natriumhypochloritlösung für 8 bis 10 Sek., wobei die Konzentration an wirksamem Chlor 6% beträgt,
- Eintauchen des Handschuhs in eine Salzsäurelösung für 8 bis 10 Sek., wobei die Konzentration an wirksamem Chlor 12% beträgt,
- wenigstens dreimaliges Wiederholen der zuletzt genannten zwei Schritte, um den gewünschten Halogenierungsgrad auf der Innenfläche zu erhalten, und
- Waschen des Handschuhs in Wasser, bis alle Chlorreste entfernt sind.

## Revendications

1. Procédé d'halogénation de gants chirurgicaux en caoutchouc naturel sur l'une et l'autre des surfaces intérieure et extérieure à des degrés différents, comportant les opérations consistant :
- à placer le gant en caoutchouc naturel sur un moule à la forme de la main,
- à plonger le gant pendant 1 à 60 secondes dans une solution d'hypochlorite de sodium, la concentration de chlore actif dans la solution étant comprise entre 0,1 et 15%,
- à plonger le gant dans une solution d'acide chlorhydrique pendant 1 à 60 secondes, la concentration de chlore actif dans la solution étant comprise entre 0,1 et 15%,
- à laver le gant à l'eau jusqu'à ce que tout le chlore résiduel soit retiré,
- à retirer le gant du moule et le retourner le dedans dehors et placer le gant sur le moule à la forme de la main,
- à plonger le gant pendant 1 à 60 secondes dans une solution d'hypochlorite de sodium, la concentration de chlore actif dans la solution étant comprise entre 1 et 15%,
- à plonger le gant dans une solution d'acide chlorhydrique pendant 1 à 60 secondes, la concentration de chlore actif dans la solution étant comprise entre 1 et 36%,
- à repéter les deux dernières opérations mentionnées au moins 3 fois de façon à obtenir sur la surface intérieure un degré d'halogénation différent de celui obtenu sur la surface extérieure et
- à laver le gant dans l'eau jusqu'à ce que tout le chlore résiduel soit retiré.

2. Procédé d'halogénation de gants chirurgicaux en caoutchouc naturel sur l'une et l'autre des surfaces intérieure et extérieure à des degrés différents, selon la revendication 1, comportant les opérations consistant :
- à placer le gant en caoutchouc naturel sur un moule à la forme de la main,
- à plonger le gant pendant 8 à 10 secondes dans une solution d'hypochlorite de sodium, la concentration de chlore actif dans la solution étant comprise entre 0,5 et 5%, de préférence égale à 1,5%,
- à plonger le gant dans une solution d'acide chlorhydrique pendant 8 à 10 secondes, la concentration de chlore actif dans la solution étant comprise entre 0,1 et 15%, de préférence égale à 0,16%,
- à laver le gant à l'eau jusqu'à ce que tout le chlore résiduel soit retiré,
- à retirer le gant du moule et le retourner le dedans dehors et placer le gant sur le moule à la forme de la main,
- à plonger le gant pendant 8 à 10 secondes dans une solution d'hypochlorite de sodium, la concentration de chlore actif dans la solution étant comprise entre 1 et 15%, de préférence égale à 6%,
- à plonger le gant dans une solution d'acide chlorhydrique pendant 8 à 10 secondes, la concentration de chlore actif dans la solution étant comprise entre 6 et 24%, de préférence égale à 12%,
- à repéter les deux dernières opérations mentionnées au moins 3 fois de façon à obtenir sur la surface intérieure un degré d'halogénation différent de celui obtenu sur la surface extérieure et
- à laver le gant dans l'eau jusqu'à ce que tout le chlore résiduel soit retiré.

3. Procédé d'halogénation de gants chirurgicaux en caoutchouc naturel sur la surface extérieure, comportant les opérations consistant :
- à placer le gant en caoutchouc naturel sur un moule approprié qui est à la forme de la main,
- à plonger le gant dans une solution d'hypochlorite de sodium pendant 8 à 10 secondes, la concentration de chlore actif dans la solution étant égale à 1,5%,
- à plonger le gant dans une solution d'acide chlorhydrique pendant 8 à 10 secondes, la concentration de chlore actif dans la solution étant égale à 0,16%,
- à laver le gant à l'eau jusqu'à ce que tout le chlore résiduel soit retiré.

4. Procédé d'halogénation de gants chirurgicaux en caoutchouc naturel sur la surface intérieure, comportant les opérations consistant :
- d'abord à retourner le gant en caoutchouc naturel le dedans dehors et placer le gant sur un moule approprié qui est à la forme de la main,
- à plonger le gant dans une solution d'hypochlorite de sodium pendant 8 à 10 secondes, la concentration de chlore actif étant égale à 6%,
- à plonger le gant dans une solution d'acide chlorhydrique pendant 8 à 10 secondes, la concentration de chlore actif étant égale à 12%,
- à repéter les deux dernières opérations mentionnées au moins 3 fois de façon à obtenir sur la surface intérieure un degré d'halogénation voulu et
- à laver le gant dans l'eau jusqu'à ce que tout le chlore résiduel soit retiré.
